# EUROPEAN PATENT APPLICATION

(11) **EP 1 495 721 A2**
(43) Date of publication of application: **12.01.2005**
(21) Application number: 04015327.2
(22) Date of filing: 30.06.2004
(51) Int. Cl.: A61B 7/00

(54) **Biological-sound data processing system, program, and recording medium**

(30) Priority: 08.07.2003 JP 2003193837
(71) Applicant: Konica Minolta Medical & Graphic Inc., Tokyo 163-0512 (JP)
(72) Inventor: Wada, Yasunori, 1 Sakura-machi Hino-shi Tokyo, 191-8511 (JP)
(74) Representative: Henkel, Feiler & Hänzel

(57) **Abstract**

A biological-sound data processing system including: a biological-sound data detecting apparatus for detecting a plurality of biological-sound data sequentially from a plurality of different detecting positions; a data processing apparatus to convert detected biological-sound data into visible data, and to correspond the each of detecting positions to at least one of detected data and visible data; and a displaying apparatus to display the visible data according to each of the detecting positions.

## Description

### FIELD OF THE INVENTION

The present invention relates to a biological-sound data processing system for detecting biological sounds and processing their data.

### BACKGROUND OF THE INVENTION

Auscultation of respiratory sounds has long been an important clinical examination and diagnosis means conducted by doctors. Although medical technology has recently progressed and advanced diagnostic equipment and data processing systems have been developed, the method of the auscultating respiratory sounds remains unchanged. As the result of diversification of medical services, the objectivity and storage of diagnostic data is a requirement in order to provide clear explanations to patients, to conduct clinical examinations in home-care or home-visit nursing care services, and to prevent or verify medical treatment errors.

However, auscultation data collected in real time ceases to exist immediately after a doctor has auscultated. Therefore, it is difficult to show this data to the patient when providing a detailed explanation to a patient, which means that it is not easy for the patient to objectively understand his/her medical condition. Furthermore, a doctor is not able to auscultate biological sounds although a nurse conveys judgment with regard to auscultation information obtained during home-visit nursing care service. Accordingly, auscultation equipment is required which allows someone other than a doctor to conduct advanced clinical examination by conducting skilled auscultation.

In the light of the above, for example, Japanese Application Patent Laid-open Publication No. 2002-165789 has disclosed a technology for detecting auscultatory sounds by a microphone, converting the sounds into digital data, and comparing the data with auscultatory sound reference data of various diseases thereby diagnosing the name of a patient's disease. In addition, Japanese Application Patent Laid-Open Announcement No. 2001-505085 has disclosed a method for measuring respiratory sounds generated by the respiratory system and identifying a given type of respiratory sound when the measured respiratory sounds meet the criteria which indicate characteristics of a given type of respiratory sound.

With regard to respiratory sound diagnosis, there is a diagnostic method for detecting respiratory sounds from a plurality of positions, analyzing the characteristics of each sound and focusing attention on sound differences according to the positions thereby giving a patient diagnosis. For example, at the lower part of the lung, normally, inspiratory sound is heard, but expiratory sound is seldom heard or its sound is diminished when it is heard. On the other hand, when there is water in the lung, sound propagation is good, which allows expiratory sound at the lower part of the lung to be heard. Therefore, when expiratory sound is heard from the lower part of the lung, diagnosis will indicate that there is a high possibility that there is water in the lung. To give accurate diagnosis, it is preferable that respiratory sounds be detected from a plurality of positions and are compared with one another.

However, when a plurality of data is displayed one at a time, it is difficult to conduct comparative diagnosis. In addition, every time the position to detect sounds is changed, for example, upper right, upper left, bottom right, and bottom left of the chest, the position to detect sounds must be specified for the processing mechanism. This is very cumbersome for busy doctors or examiners. As a result, an optimal medical diagnosis support system is required.

The Japanese Application Patent Laid-Open Announcement No. 2001-505085 describes how to analyze the characteristics of respiratory sounds, but it does not give detailed description of the display method.

An objective of the present invention is to provide a biological-sound signal processing system for detecting biological sounds from a plurality of different positions, and displaying diagnostic information based on the obtained sound data, thereby increasing diagnostic accuracy.

### SUMMARY OF THE INVENTION

To achieve the aforementioned objective:
1. A biological-sound signal processing system for processing biological-sound data detected from within the human body by a biological-sound detection means comprises a signal processing means to convert biological-sound data detected sequentially from a plurality of different positions into visible data, and a display means to display such data by relating the data to each position from which the data has been detected.
   Herein, "to convert data into visible data" means to process data so that it can be visually recognized in graphs, tables, or the like.
   According to a biological-sound processing system described in the above item 1, the system converts biological-sound data detected sequentially from a plurality of different positions into visible data and displays the data by relating it to each position from which the data has been detected. Therefore, this system makes it possible for a user to easily compare a plurality of biological-sound data thereby increasing diagnostic accuracy.
2. A biological-sound signal processing system for processing biological-sound data detected from within the human body by a biological-sound detection means comprises a signal processing means to analyze biological-sound data detected sequentially from a plurality of different positions and retrieve the characteristics of the biological sound, type of candidate disease, or the probability of diagnosis of the disease, and a display means to display the retrieved characteristics of the biological sound, type of candidate disease, or the probability of diagnosis of the disease.
   Herein, an analysis technique based on the FFT (Fast Fourier Transformation) or wavelet analysis or an analysis technique for voice recognition can apply to the biological-sound data analysis method.
   According to a biological-sound processing system described in item 2, the system displays the characteristics of the biological sound obtained by analyzing biological-sound data detected sequentially from a plurality of different positions, type of candidate disease, or the probability of diagnosis of the disease, thereby increasing diagnostic accuracy.
3. A biological-sound processing system according to item 2 refers to the difference between the detected biological sounds according to their detection positions when the biological-sound data is analyzed.
   According to a biological-sound processing system described in item 3, the system retrieves the characteristics of the biological sound, type of candidate disease, or the probability of diagnosis of the disease by referring to the difference between the detected biological sounds thereby increasing diagnostic accuracy.
4. A biological-sound signal processing system described in item 2 analyzes the detected biological-sound data by comparing the data with case data which has been stored.
   According to a biological-sound processing system described in item 4, the system retrieves the characteristics of the biological sound, type of candidate disease, or the probability of diagnosis of the disease by comparing the detected biological-sound data with case data which has been stored, thereby increasing diagnostic accuracy.
5. In a biological-sound signal processing system according to any one of items 1 through 4, a plurality of different positions to detect sounds are specified in a prescribed sequence and the detected biological-sound data is attributed to each position according to the specified sequence.
   According to a biological-sound processing system described in item 5, the system attributes detected biological-sound data to each position according to the specified sequence, which eliminates the time to input data of the positions from which sounds have been detected, thereby reducing the time to detect biological sounds.
6. In a biological-sound signal processing system according to item 5, more than one sequence pattern for a plurality of different positions to detect sounds is selectively specified, and the sequential order of the positions to detect sounds is determined by selecting a prescribed sequence pattern.
   According to a biological-sound signal processing system described in item 6, it is possible to detect biological-sound data in a sequential order which is convenient for a user.
7. In a biological-sound signal processing system according to any one of items 1 through 4, the biological-sound detection means has a position detection means to detect the position on the human body at which the biological-sound detection means is located.
8. In a biological-sound signal processing system according to item 7, the position detection means is an acceleration sensor.
   In a biological-sound signal processing system according to item 7 or 8, the biological-sound detection means has a position detection means to detect its position on the human body, thereby making it possible to relate a position to the biological-sound data detected at that position.
9. In a biological-sound signal processing system according to any one of items 1 through 8, the biological-sound detection means has an instruction means to transmit operation instruction signals to the signal processing means.
10. In a biological-sound signal processing system according to item 9, the operation instruction signals include an instruction for starting or ending the biological-sound detection operation, instruction for changing a sequential order of the positions to detect sounds, or instruction for discarding detected data.
11. In a biological-sound signal processing system according to item 10, the instruction means transmits an instruction signal to start or end the biological-sound detection operation in conjunction with an operation of the biological-sound detection means, such as coming in contact with the human body, approaching the human body, or moving away from the human body.
   According to a biological-sound signal processing system described in any one of items 9 through 11, the biological-sound detection means that has an instruction means to transmit operation instruction signals to the signal processing means enables a user to transmit an operation instruction signal from the biological-sound detection means.
12. A biological-sound signal processing system according to any one of items 1 through 11 further comprises a storage means to store detected biological-sound data, wherein the signal processing means retrieves a plurality of biological-sound data detected from the same person at different time periods from biological-sound data stored in the storage means and compares the old and new data, and the display means displays the comparative result of the old and new biological-sound data.
13. In a biological-sound signal processing system according to item 12, the comparative result of the old and new biological-sound data indicates that there has been a change of the characteristics of the biological sound, or a change of candidate disease.
   According to a biological-sound signal processing system described in item 12 or 13, it is possible to compare a plurality of biological-sound data detected from the same person at different time periods, thereby making it possible to quantitatively observe aggravation or improvement of a disease, or the effects of medical treatment or medication.
14. In a biological-sound signal processing system according to any one of items 1 through 13, the biological-sound detection means has a wireless transmission means to wirelessly transmit the detected biological-sound data to the signal processing means.

When a long conductor cable is used to convey signals from the biological-sound detection means to the signal processing means, the conductor cable could be brushed or impacted by obstacles, thereby conveying vibration to the biological-sound detection means and causing noise.

According to a biological-sound signal processing system described in item 14, biological-sound data is wirelessly transmitted from the biological-sound detection means to the signal processing means, which does not require a cable for conveying signals between the biological-sound detection means and the signal processing means, thereby reducing noise in the biological-sound data.

Various kinds of existing means can apply to the wireless transmission method. When considering medical sites, it is preferable that the wireless transmission method use an infrared ray or the like to avoid failure of medical equipment due to electromagnetic waves or use an electromagnetic wave, such as Bluetooth. However, an infrared ray can be blocked by obstacles located between the transmission means and the receiving,means, resulting in reception failure. In that situation, applicable methods are: a method in which the signals are received at a location where there is few obstacles between the biological-sound detection means and the receiving means and the signals are then transferred to the signal processing means, or a method in which signals are received by a plurality of receiving means, selected, and synthesized.

### BRIEF DESCRIPTION OF THE DRAWINGS

FIG. 1 is a schematic diagram of a respiratory-sound signal processing system 1 according to an embodiment of the present invention.
FIG. 2 is a block diagram showing functional configuration of the respiratory-sound signal processing system 1.
FIG. 3(a) to Fig. 3(c) explains the structure of a microphone 2.
FIG. 4 explains wireless transmission of respiratory-sound data.
FIG. 5 is a flow chart showing the respiratory-sound signal processing conducted by a respiratory-sound signal processing system 1.
FIG. 6 explains a method to determine a sequential order to detect respiratory-sound data.
FIG. 7 shows an example how respiratory-sound data detected from each position is displayed.
FIG. 8 explains how the location of a microphone is automatically detected in modification 1.
FIG. 9 explains how an instruction for starting or ending the respiratory-sound data detection operation is automatically provided in modification 2.

### DETAILED DESCRIPTION OF THE PREFERRED EMBODIMENT

Hereafter, an embodiment of the present invention will be described in detail with reference to the drawings.

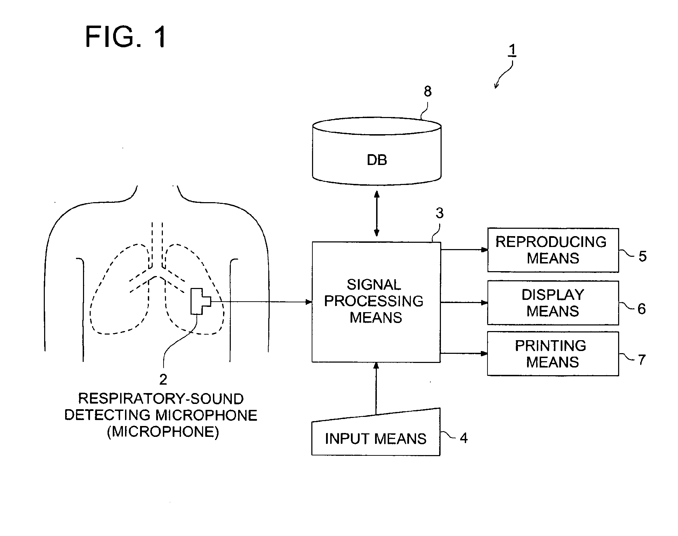

FIG. 1 is a schematic diagram of a respiratory-sound signal processing system 1 according to an embodiment of the present invention. In this embodiment, respiratory sound (or lung sound) is used as an example of biological sound; however, other biological sound (e.g., sound of the heart or digestive system) can apply to the present invention.

As shown in FIG. 1, the respiratory-sound signal processing system 1 consists of a microphone for detecting respiratory sounds (hereafter referred to as microphone) 2, signal processing means 3, input means 4, reproducing means 5, display means 6, printing means 7, and a database 8.

Respiratory-sound data is detected from the human body by means of the microphone 2 and its signal is processed by the signal processing means 3 according to an instruction provided by the input means 4 by referring to the data stored in the database 8. The processed result is then outputted by the reproducing means 5, display means 6, or printing means 7.

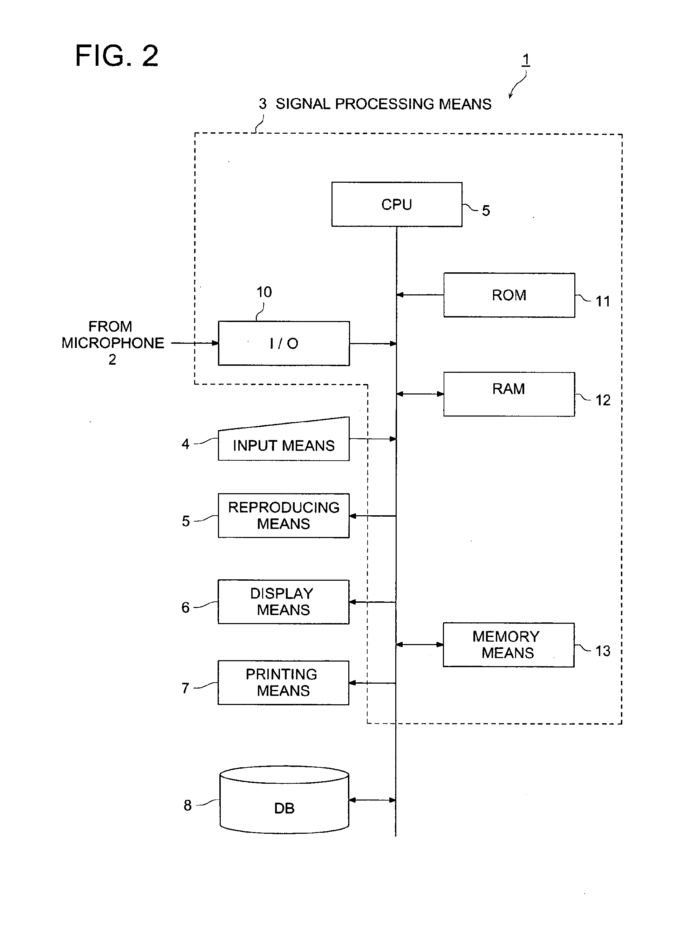

FIG. 2 is a block diagram showing functional configuration of the respiratory-sound signal processing system 1.

The microphone 2 detects respiratory sounds sequentially from a plurality of different positions in the human body and outputs electrical signals (respiratory-sound data). Various types of microphones 2 are available. For example, an electric capacitor microphone or piezo element is used.

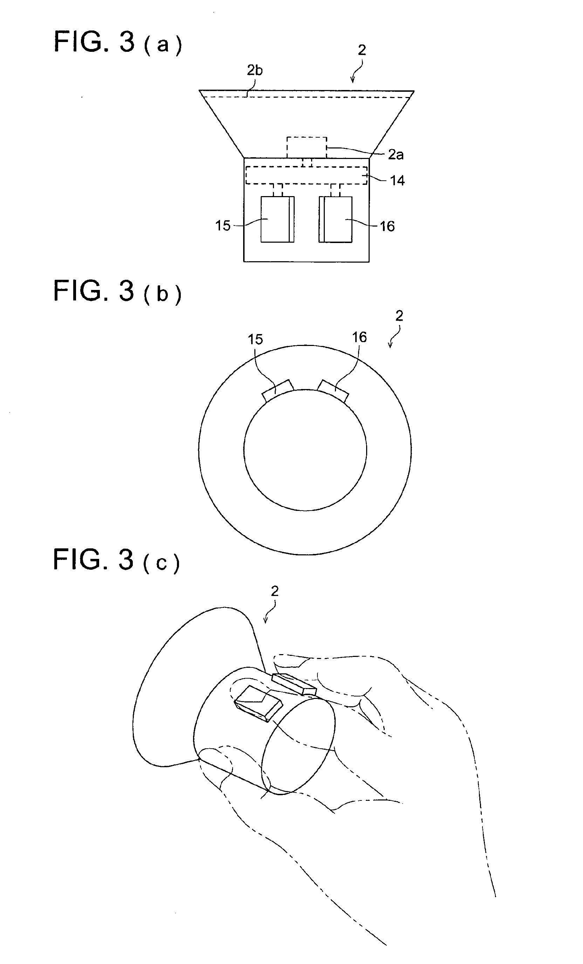

FIG. 3(a) and Fig. 3(b) are a top view of the microphone 2 and a front view, respectively. As shown in FIG. 3(a) and Fig. 3(b), the microphone 2 comprises a capacitor microphone 2a, diaphragm 2b, and switches 15 and 16 to transmit operation instruction signals to a wireless transmission means 14 and a signal processing means 3. As shown in FIG. 3(c), by placing the forefinger on switch 15 and the middle finger on switch 16, it is possible to press switches 15 and 16 while operating the microphone 2 with one hand.

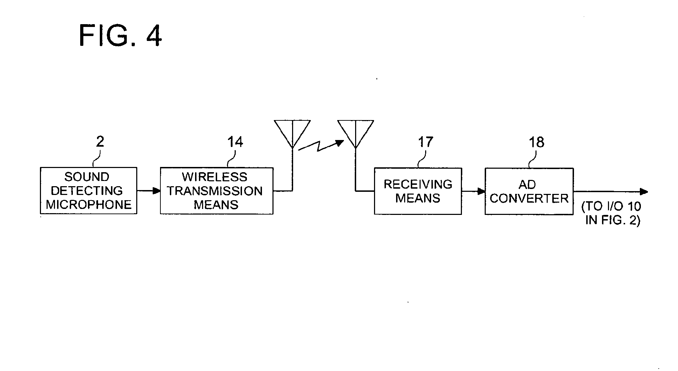

As shown in FIG. 4, both respiratory-sound data (electrical signal) detected by the microphone 2 and instruction signals from switches 15 and 16 are converted into a radio wave by the wireless transmission means 14 and transmitted from an antenna. The radio wave is so weak that it does not affect other medical equipment. The radio wave transmitted from the wireless transmission means 14 is received by the receiving means 17 and demodulated into an electrical signal. The electrical signal is then converted into a digital signal by an AD converter 18, and outputted to the I/O 10 of the signal processing means 3 shown in FIG. 2.

An operation instruction signal is transmitted by a user pressing switches 15 and 16. According to a pattern indicated by how switches 15 and 16 were pressed and how many times the switches were pressed, instructions are transmitted for starting or ending the respiratory-sound detection operation, changing the sequential order of the positions to detect sounds, or discarding the detected data. For example, instructions to be transmitted could be: pressing switch 15 once means to start the respiratory-sound detection operation, pressing switch 15 twice in quick succession means to detect respiratory sounds again and discard the previously detected data, and pressing switch 16 once means to skip the respiratory-sound detection operation at that position and move to the next position.

As shown in FIG. 2, the signal processing means 3 comprises the CPU (Central Processing Unit) 9, I/O 10, ROM (Read Only Memory) 11, RAM (Random Access Memory) 12, and a memory means 13. A PC (Personal Computer) or PDA (Personal Digital Assistants: mobile device) can be used as a signal processing means 3.

According to an instruction inputted by the input means 4, the CPU 9 selects a specified program from various programs stored in the ROM 11, develops the program in the work area of the RAM 12, executes various processing operations in cooperation with the aforementioned program, and then stores the processed result in a prescribed area of the RAM 12.

The I/O 10 receives respiratory-sound data outputted from the microphone 2 and outputs the data to the CPU 9.

The ROM 11 is made up of a nonvolatile semiconductor memory. The ROM 11 stores programs for the respiratory-sound signal processing system 1 which are executed by the CPU 9 as well as various kinds of data.

The RAM 12 is made up of a rewritable semiconductor element. The RAM 12 is a memory medium in which data is temporarily stored. In the RAM 12, different areas are formed, such as a program area in which programs executed by the CPU 9 are developed, and a data area in which data inputted by the input means 4 and various results processed by the CPU 9 are stored.

The memory means 13 has a HDD (Hard Disk Drive) which stores respiratory-sound data detected by the microphone 2, processed data, and sequence patterns for the positions to detect respiratory-sound data.

The input means 4 includes a keyboard consisting of alphanumeric input keys, function keys, and a pointing device such as a mouse. The input means outputs to the CPU 9 a pressing signal generated when the keyboard's key is pressed and an operation signal when the mouse is operated.

The reproducing means 5 has a speaker or headphone, and reproduces sound of the respiratory-sound data detected by the microphone 2.

The display means 6 has a CRT (Cathode Ray Tube), liquid crystal display, or plasma display. It displays respiratory-sound data which has been converted into visible data by the signal processing means 3 and the characteristics of the biological sound, type of candidate disease, or the probability of diagnosis of the disease which has been retrieved by the signal processing means 3.

The printing means 7 prints out on the recording paper respiratory-sound data which has been converted into visible data by the signal processing means 3 and the characteristics of the biological sound, type of candidate disease, or the probability of diagnosis of the disease which has been retrieved by the signal processing means 3.

The database 8 stores case data for diseases which can be diagnosed from the respiratory sounds, and types of candidate diseases. The database 8 also stores respiratory-sound data detected in the past and its analytic results.

Next, operations of the respiratory-sound signal processing system 1 according to this embodiment will be described.

Description of the operations premises that programs for executing operations, described in the flow chart, are stored in the ROM 11 in the form of a program code which the CPU 9 of the respiratory-sound signal processing system 1 can read and the CPU 9 sequentially executes operations according to the program code.

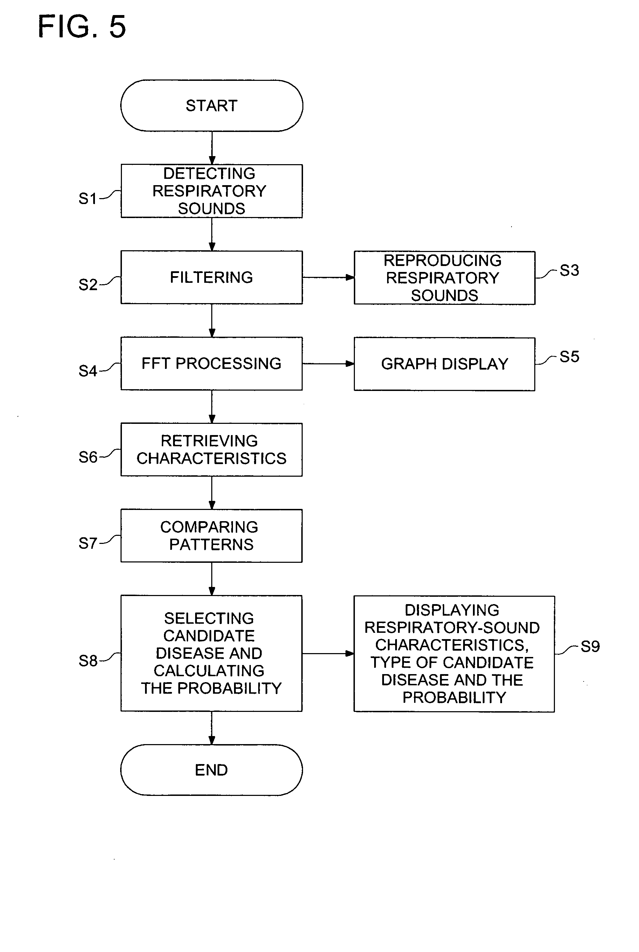

FIG. 5 is a flow chart showing the respiratory-sound signal processing conducted by a respiratory-sound signal processing system 1.

First, a microphone 2 detects respiratory-sound data sequentially from a plurality of different positions in the human body (Step S1), and the respiratory-sound data is stored in a memory means 13. The sequential order to detect respiratory-sound data can be selected from the sequence patterns for a plurality of positions to detect respiratory sounds stored in the memory means 13, and a user selects one of the sequence patterns for the positions to detect respiratory sounds. According to the selected sequence pattern, respiratory-sound data is detected, and the respiratory-sound data is then related to each position from which the sound was detected according to the selected sequence.

Herein, switches 15 and 16 provided on the microphone 2 transmit an operation instruction signal to instruct the respiratory-sound detection operation to begin or cease. It is possible to end the respiratory-sound detection operation by detecting characteristic sound generated when the microphone 2 is moved away from the human body. Switches 15 and 16 also transmit an instruction to change the sequential order to detect respiratory sounds from different positions, or an instruction to discard detected respiratory-sound data when the detection operation failed.

Next, the detected respiratory-sound data is filtered to remove noise in the respiratory-sound data (Step S2). According to an instruction by the input means 4, the respiratory-sound data which by now is noise free is transformed back into a sound by means of a speaker or headphone (reproducing means 5) (Step S3).

Next, the noise-free, respiratory-sound data is processed with the FFT processing (Step S4). According to an instruction by the input means 4, the FFT processed result is converted into visible data, related to each detection position from which the sound was detected, and displayed in a graph by the display means 6 (Step S5). It is possible to display numeric values on which a diagnosis is based.

Next, differences of amplitude or tone color of the respiratory sounds according to detection positions are referred to, respiratory-sound data detected from a plurality of different positions is analyzed, and the characteristics of the respiratory sounds are retrieved (Step S6). The obtained respiratory-sound data and the characteristics of the respiratory sounds are compared with disease case data stored in the database 8 (Step S7), and then a candidate disease is selected, and the probability of diagnosis of the disease is calculated (Step S8).

Herein, how the probability of diagnosis of the disease is calculated will be described.

The probability of the disease can be calculated based on the clinical statistical data.

For example, if there is statistical data indicating that a certain type of adventitious sound (crackle) is detected from x % of patients who have disease A, the probability that a patient who has adventitious sound suffers from disease A is x %.

If there is statistical data indicating that a certain number of specific adventitious sounds is detected from one respiration of each patient who has disease B, when the number of adventitious sounds detected from one respiration of a patient is n or more, it is possible to obtain the probability that the patient is diagnosed with disease B. A large number of clinical data has been reported concerning the relationship between the number of adventitious sounds and pneumonia, making it possible to provide reliable probability.

In addition to the number of adventitious sounds, frequency, occurrence timing and time duration of wheeze can become useful parameters for identifying disease and the level of seriousness.

Other parameters can be obtained by retrieving an amount of characteristics by means of waveform analysis. When diagnosing a case in the clinical scene by means of auscultation, it is useful to provide name of candidate disease and probability of the disease according to the statistical data as well as to provide parameters.

Furthermore, the present invention can be used for the probability calculation in combination with other diagnosis methods including a diagnosis using an electromagnetic wave and sonic wave (radiographing, CT, MRI and endoscope), an inspection of specimen and an inspection of DNA. For example, if the probability of a certain disease is X % according to the diagnostic imaging system and the probability of a certain disease independently calculated by the present invention is Y %, the compound result of the probability of a certain disease can be calculated by the equation: 100 - (100 - x) X (100 - y). This enables users to identify disease with a higher probability than individual diagnosis.

The auscultatory sound is generated by dynamic movement of the lungs and is considered different information from image information which shows the diseased part by means of X-ray photographing or MRI using electromagnetic waves. Accordingly, it is useful to combine auscultation with such methods.

If biological sounds are detected from a plurality of positions as shown in this embodiment of the present invention, it is possible to determine diagnosis by associating position information of the diseased part obtained by the diagnostic imaging system with sound information generated around the diseased part. Consequently, this combined method is most useful.

After that, according to an instruction by the input means 4, the display means 6 displays the characteristics of the respiratory sounds, type of candidate disease, or the probability of diagnosis of the disease (Step S9). For example, the display means 6 shows like this: intermittent La-sounds are heard from the upper part of the left lung, and the intensity is level 5; a loud inspiratory sound is heard from the lower part of the left lung; a candidate disease diagnosed based on an intermittent sound pattern could be hypersensitivity alveolitis 70%, or pneumonia 5%. Respiratory-sound data and analytic results are stored in the database 8.

This completes the respiratory-sound signal processing.

Next, specific procedures for detecting respiratory sounds will be described below.

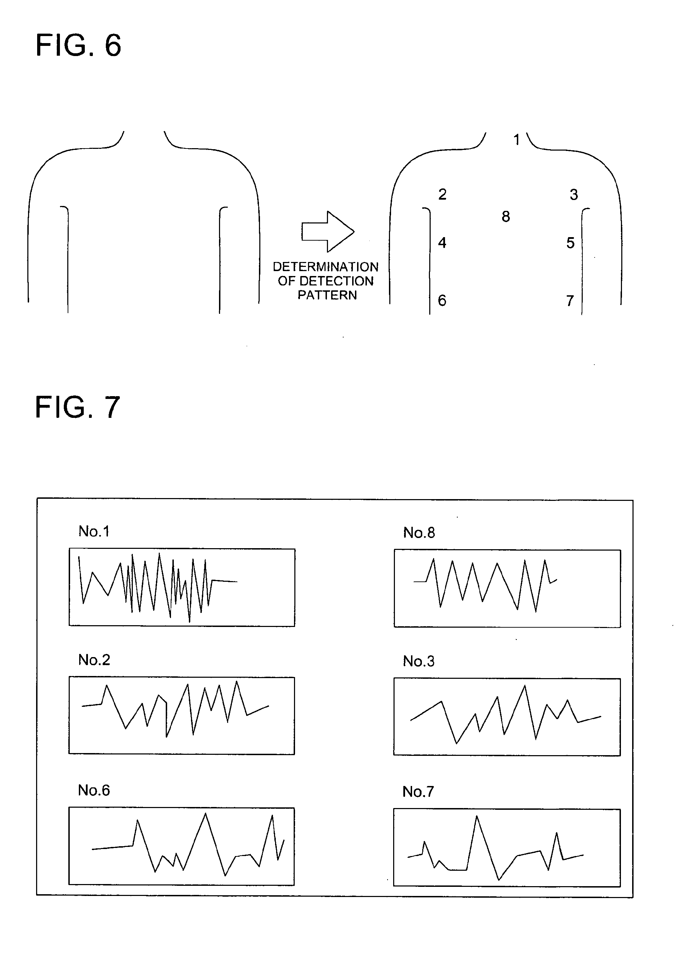

The sequential order to detect respiratory-sound data is determined by a user marking positions according to the desirable sequence in the drawing of the human body shown on the display means 6, as shown in FIG. 6. For example, in FIG. 6, the sequential order to detect respiratory sounds is specified like this: 1 patient's neck, 2 upper part of the right lung, 3 upper part of the left lung, 4 middle part of the right lung, 5 middle part of the left lung, 6 lower part of the right lung, 7 lower part of the left lung, and 8 heart.

Furthermore, the position of the graph to be displayed on the screen showing respiratory-sound data detected from each position is specified. FIG. 7 shows an example how respiratory-sound data detected from each position is displayed. To easily recognize the sound difference between the right lung and the left lung, it is preferable that the left and right lungs be vertically or horizontally positioned on the screen. Type of graph, such as a graph showing sound intensity change over time, or a graph obtained by a sound spectrogram or wavelet conversion, can be selected according to need.

By pressing switch 15 of the microphone 2 on detection position 1 on the human body, the start of the respiratory-sound detection operation is recognized and data sampling is started, and simultaneously the display means 6 starts to display the data in a graph. When moving the microphone 2 away from the human body at the end of the respiratory-sound detection operation, a characteristic sound generated when the microphone 2 is moved away from the human body is detected, thereby ending the respiratory-sound detection operation at position 1. Then, the data is stored and the system enters into a stand-by mode for detecting sound at position 2.

By pressing switch 15 of the microphone 2 on position 2 on the human body, the start of the respiratory-sound detection operation is recognized and data sampling is started, and simultaneously the display means 6 starts to display the data in a graph. Herein, if switch 15 is pressed twice in quick succession, the system recognizes that a redetection is instructed, and the system discards the sampled data and then enters into a stand-by mode for detecting sound again at position 2. By pressing switch 15 again, the start of the respiratory-sound detection operation is recognized and data sampling is started, and simultaneously the display means 6 starts to display the data in a graph. When moving the microphone 2 away from the human body at the end of the respiratory-sound detection operation, a characteristic sound generated when the microphone 2 is moved away from the human body is detected, thereby ending the respiratory-sound detection operation at position 2. Then, the data is stored and the system enters into a stand-by mode for detecting sound at position 3.

Similarly, by pressing switch 16 after the detection at position 3 has been finished, the system skips the respiratory-sound detection operation at position 4 and enters into a stand-by mode for detecting sound at position 5. By pressing switch 16 again, the system skips the respiratory-sound detection at position 5 and enters into a stand-by mode for detecting sound at position 6. After the respiratory-sound detection operations have been finished at positions 6,7 and 8, it is possible to analyze sound differences between the right and left lungs at positions 2 and 3, and 6 and 7. It is also possible to analyze sound differences between the upper and lower parts in the lung at positions 2 and 6, and 3 and 7.

According to the respiratory-sound signal processing system 1 shown in this embodiment, respiratory-sound data detected sequentially from a plurality of different positions is converted into visible data and displayed by being related to each position from which sound has been detected. Consequently, this system enables a user to easily compare a plurality of biological-sound data thereby increasing diagnostic accuracy.

Furthermore, the system analyzes respiratory-sound data detected sequentially from a plurality of different positions and displays the retrieved characteristics of the respiratory sound, type of candidate disease, or the probability of diagnosis of the disease, thereby increasing diagnostic accuracy.

The system attributes detected respiratory-sound data to each detection position according to a specified sequence. This eliminates the time to input data of the detection positions thereby reducing the time to detect respiratory sounds. Furthermore, because the user can select a sequence pattern for detecting sounds from a plurality of different positions, it is possible to detect respiratory-sound data in a sequential order which is convenient for the user.

Furthermore, the microphone 2 has switches 15 and 16 for transmitting operation instruction signals to the signal processing means 3; therefore, a user can transmit operation instruction signals from the microphone 2 while holding the microphone 2.

Furthermore, because respiratory-sound data is wirelessly transmitted from the microphone 2 to the signal processing means 3, a signal cable which connects the microphone 2 to the signal processing means 3 is unnecessary, thereby reducing noise in the respiratory-sound data. It is also possible to avoid complicated cable routing at a medical treatment site thereby facilitating the use of the microphone 2.

Moreover, it is possible to select, from the respiratory-sound data stored in the database 8, a plurality of respiratory-sound data detected from the same person at different time periods and compare the data, and to display a change of the characteristics of the respiratory sound, or a change of candidate disease on the display means 6. Conventionally, the comparison of respiratory sounds could be done only by the doctor who made the auscultation and relied on memory and past experience. Comparison is impossible for a doctor who did not make the original auscultation. By storing a patient's previous data, it is possible to quantitatively observe the aggravation or improvement of a disease, or the effects of medical treatment or medication.

### <Modification 1>

As modification 1, how the location of a microphone is automatically detected will be explained.

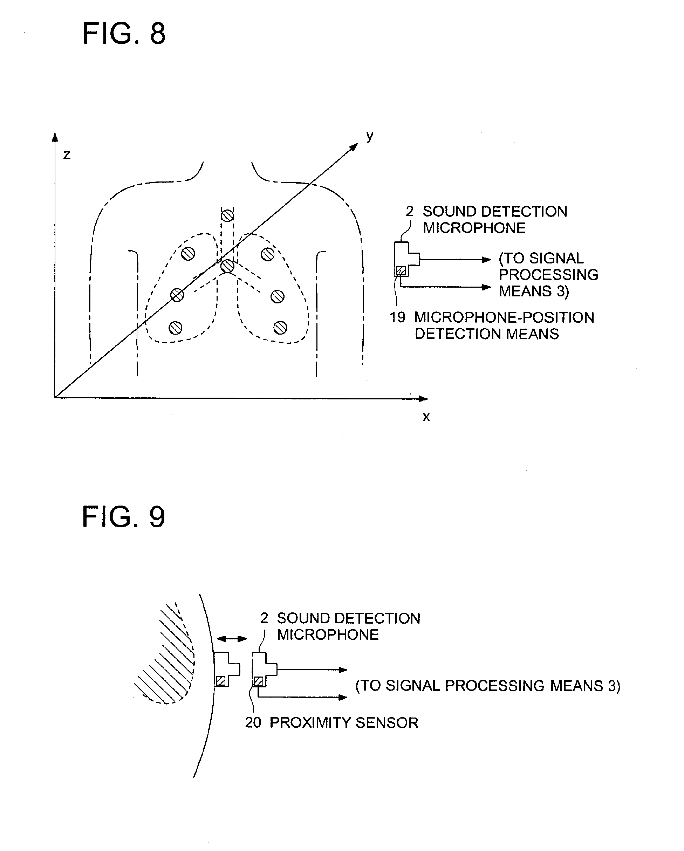

In the aforementioned embodiment, respiratory-sound data is detected according to a prescribed sequence pattern, and each detection position is identified according to the sequential order. Yet, as shown in FIG. 8, it is possible to provide a microphone-position detection means 19 to detect the location of the microphone 2 on the human body.

For example, an acceleration sensor is used as a microphone position detection means 19. By integrating the acceleration detected by an acceleration sensor, the speed can be obtained, and by integrating the value again, the traveling distance can be obtained. Thus, position information can be obtained from those values. For example, Utility Model Gazette No. Hei 06-43516 discloses the technology to detect a vehicle's traveling distance. Japanese Application Patent Laid-open Publication No. 2002-44778 also discloses the technology which obtains a microphone's position information in a concert hall and prevents audio feedback. As a microphone position detection means 19 moves, a position of the microphone to detect respiratory sounds on the human body is detected.

Because a microphone 2 has a microphone position detection means 19 to detect the location of the microphone on the human body, it is possible to relate a detection position to respiratory-sound data which has been detected from the position.

Moreover, the microphone position detection means can be attached to a microphone user's hand instead of being attached to the microphone 2.

### <Modification 2>

As modification 2, how an instruction for starting or ending the respiratory-sound data detection operation is automatically provided will be explained.

In the aforementioned embodiment, switches 15 and 16 are used as instruction means to transmit operation instruction signals to the signal processing means 3, and an operation instruction signal is transmitted by pressing switches 15 and 16. However, as shown in FIG. 9, a proximity sensor 20 can be used as an instruction means.

As a proximity sensor 20, for example, an optical sensor is used. As shown in FIG. 9, when a microphone 2 comes in contact with the human body, a ray of light entering into an optical sensor is blocked; therefore, setting is made so that the respiratory-sound data detection operation starts at that instance. When the microphone 2 is moved away from the human body, a ray of light enters into an optical sensor; therefore, setting is made so that the respiratory-sound data detection operation ends at that instance.

A proximity sensor 20 transmits an instruction signal for starting or ending the respiratory-sound data detection operation in conjunction with the microphone 2 coming in contact with the human body, approaching the human body, or moving away from the human body. Accordingly, it is possible to automatically instruct the signal processing means 3 to change operations.

A touch sensor or body sensor can be used as a means for detecting that the microphone 2 comes in contact with the human body, approaches the human body, or is moved away from the human body.

Descriptions in the aforementioned embodiment and altered examples are preferred examples of the respiratory-sound signal processing system according to the present invention and the present invention is not limited to those examples. Each of the components which make up a respiratory-sound signal processing system 1 and each specific operation can be changed within the range that conforms to the concept of the present invention.

Furthermore, it is also preferable that respiratory-sound data and analytic results be stored in an electronic medical chart. Since data storage in the electronic medical chart enables medical data to be centrally managed, it can be utilized as a database to relate a disease to respiratory sound. Moreover, the data storage contributes to retrieving the sound or pattern characteristic to a disease, and therefore it can be used as a data source to increase accuracy of diagnosis which utilizes the present invention.

## Claims

1. A biological-sound data processing system comprising:
a biological-sound data detecting apparatus for detecting a plurality of biological-sound data sequentially from a plurality of different detecting positions of an object;
a data processing apparatus to convert detected biological-sound data into visible data, and to correspond the each of detecting positions to at least one of detected data and visible data; and
a displaying apparatus to display the visible data according to each of the detecting positions.

2. The biological-sound data processing system of claim 1, wherein the plurality of different detecting positions are specified in a prescribed sequence, and
wherein the data processing apparatus correspond the detected biological-sound data with the specified sequence.

3. The biological-sound data processing system of claim 2, wherein the specified sequence is selected from at least one of sequence patterns for detecting biological-sound data on a plurality of different positions sequentially.

4. The biological-sound data processing system of claim 1, wherein the biological-sound data detecting apparatus comprises a position detecting device to detect the position on a object to be detected at which the biological-sound data detecting apparatus is located.

5. The biological-sound data processing system of claim 4, wherein the position detecting device comprising an acceleration sensor.

6. The biological-sound data processing system of claim 1, wherein the biological-sound data detecting apparatus comprises an instructing device to transmit operational instruction data to the data processing apparatus.

7. The biological-sound data processing system of claim 6, wherein the operational instruction data includes at least one of a starting instruction data for starting the biological-sound detecting operation, an terminating instruction data for terminating the biological-sound detecting operation, an changing instruction data for changing a sequential order of the positions to be detected and an deleting instruction data for deleting detected data.

8. The biological-sound data processing system of claim 7, wherein the instructing device transmits at least one of the starting instruction data and the terminating instruction data, in conjunction with the operation of the biological-sound data detecting apparatus including at least one of contacting with, approaching and moving away from the object.

9. The biological-sound data processing system of claim 1, further comprising:
a storing device to store the detected biological-sound data, wherein the data processing apparatus retrieves a plurality of biological-sound stored-data detected from the same object at different time periods from biological-sound data stored in the storing device, and compares the biological-sound stored-data and the detected biological-sound data, and wherein the displaying apparatus displays the comparative result compared by the data processing apparatus.

10. The biological-sound data processing system of claim 9, wherein the comparative result indicates at least one of a change of the characteristics of the biological-sound data and a change of candidate disease.

11. The biological-sound data processing system of claim 1, wherein the biological-sound data detecting apparatus comprising a wireless transmission device to wirelessly transmit the detected biological-sound data to the data processing apparatus.

12. The biological-sound data processing system of claim 1, wherein the data processing apparatus analyzes the detected biological-sound data for extracting a risk whether detected biological-sound data corresponds with a candidate disease the disease or not, and
wherein the displaying apparatus to display information according to the risk.

13. A biological-sound data processing system comprising:
a biological-sound data detecting apparatus for detecting a plurality of biological-sound data of an object;
a data processing apparatus to analyze detected biological-sound data for extracting a risk whether detected biological-sound data corresponds with a candidate disease the disease or not; and
a displaying apparatus to display information according to the risk.

14. The biological-sound data processing system of claim 13, wherein the data processing apparatus analyzes on base of at least a difference of a plurality of detected biological-sound data according to each of the detecting positions of the object.

15. The biological-sound data processing system of claim 13, wherein the data processing apparatus analyzes detected biological-sound data by comparing the data with case data which has been stored.

16. The biological-sound data processing system of claim 13, wherein the biological-sound data detecting apparatus detects the plurality of biological-sound data sequentially from a plurality of different detecting positions.

17. The biological-sound data processing system of claim 16, wherein the plurality of different positions are specified in a prescribed sequence, and
wherein the data processing apparatus correspond the detected biological-sound data with the specified sequence.

18. The biological-sound data processing system of claim 17, wherein the specified sequence is selected from at least one of sequence patterns for detecting biological-sound data on a plurality of different positions sequentially.

19. The biological-sound data processing system of claim 13, wherein the biological-sound data detecting apparatus comprises a position detecting device to detect the position on a object to be detected at which the biological-sound data detecting apparatus is located.

20. The biological-sound data processing system of claim 19, wherein the position detecting device comprising an acceleration sensor.

21. The biological-sound data processing system of claim 13, wherein the biological-sound data detecting apparatus comprises an instructing device to transmit operational instruction data to the data processing apparatus.

22. The biological-sound data processing system of claim 21, wherein the operational instruction data includes at least one of a starting instruction data for starting the biological-sound detecting operation, an terminating instruction data for terminating the biological-sound detecting operation, an changing instruction data for changing a sequential order of the positions to be detected and an deleting instruction data for deleting detected data.

23. The biological-sound data processing system of claim 22, wherein the instructing device transmits at least one of the starting instruction data and the terminating instruction data in conjunction with the operation of the biological-sound data detecting apparatus including at least one of contacting with, approaching and moving away from the object.

24. The biological-sound data processing system of claim 13, further comprising:
a storing device to store detected biological-sound data; wherein the data processing apparatus retrieves a plurality of biological-sound stored-data detected from the same object at different time periods from biological-sound data stored in the storing device, and compares the biological-sound stored-data and the detected biological-sound data, and
wherein the displaying apparatus displays the comparative result compared by the data processing apparatus.

25. The biological-sound data processing system of claim 13, wherein the comparative result indicates at least one of a change of the characteristics of the biological-sound data and a change of candidate disease.

26. The biological-sound data processing system of claim 13, wherein the biological-sound data detecting apparatus comprising a wireless transmission device to wirelessly transmit the detected biological-sound data to the data processing apparatus.

27. The biological-sound data processing system of claim 13, wherein the data processing apparatus analyzes the detected biological-sound data in combination with other diagnosis methods for extracting the risk.

28. A computer program to control a computer to function as a biological-sound data processor, wherein the biological-sound data processor comprises:
a detecting function for detecting a plurality of biological-sound data sequentially from a plurality of different detecting positions;
a data processing function for converting detected biological-sound data into visible data, and for corresponding the each of detecting positions to at least one of detected data and visible data; and
a displaying function for displaying the visible data according to each of the detecting positions.

29. A recording medium, which comprises a program to control a computer to function as a biological-sound data processor, wherein the biological-sound data processor comprising:
a detecting function for detecting a plurality of biological-sound data sequentially from a plurality of different detecting positions;
a data processing function for converting detected biological-sound data into visible data, and for corresponding the each of detecting positions to at least one of detected data and visible data; and
a displaying function for displaying the visible data according to each of the detecting positions.

30. A computer program to control a computer to function as a biological-sound data processor, wherein the biological-sound data processor comprises:
a biological-sound data detecting function for detecting a plurality of biological-sound data;
a data processing function to analyze the detected biological-sound data for extracting a risk whether detected biological-sound data corresponds with a candidate disease the disease or not; and
a displaying function for displaying information according to the risk.

31. A recording medium, which comprises a program to control a computer to function as a biological-sound data processor, wherein the biological-sound data processor comprising:
a biological-sound data detecting function for detecting a plurality of biological-sound data;
a data processing function to analyze the detected biological-sound data for extracting a risk whether detected biological-sound data corresponds with a candidate disease the disease or not; and
a displaying function for displaying information according to the risk.
